# EUROPÄISCHE PATENTSCHRIFT

(11) **EP 2 792 386 B1**
(45) Veröffentlichungstag und Bekanntmachung des Hinweises auf die Patenterteilung: **12.06.2019**
(21) Anmeldenummer: 13163999.9
(22) Anmeldetag: 16.04.2013
(51) Int. Cl.: A61N 1/40

(54) **Behandlungsliege**
Treatment bed
Lit de soin

(43) Veröffentlichungstag der Anmeldung: 22.10.2014
(73) Patentinhaber: Celsius42 GmbH, 52249 Eschweiler (DE)
(72) Erfinder: Glomb, Lars, 51147 Köln (DE)
(74) Vertreter: Claessen, Rolf

(56) Entgegenhaltungen:
- EP-A2- 0 336 370
- DE-A1- 3 306 391
- US-A- 2 502 865
- US-A- 4 140 130

## Beschreibung

Die Erfindung betrifft eine Behandlungsliege geeignet für die Anwendung der Hyperthermie sowie ein Elektrodenmodul zur Verwendung mit der Behandlungsliege.

Verschiedene Krankheiten und insbesondere Krebs werden unter anderem thermisch behandelt. Dabei wird nach Möglichkeit innerhalb des Körpergewebes eine bestimmte Temperatur eingestellt. Hierbei wird beispielsweise die Elektro-Hyperthermie eingesetzt, die sich zu Nutze macht, dass extrazelluläre Flüssigkeit am Tumorgewebe Frequenzen um etwa 10 MHz besonders gut absorbieren kann. Zu beiden Seiten des Gewebes werden also bei dieser Therapie korrespondierende Elektroden angeordnet und ein elektromagnetisches Feld in einem Bereich von 8 bis 15 MHz angelegt. Hierbei wird besonders das Tumorgewebe erwärmt.

Diese Therapie wird üblicherweise so durchgeführt, dass die Patienten auf eine Liege gelegt werden, in der sich eine Elektrode befindet. Die korrespondierende Elektrode wird mittels eines Arms von oben auf den Patienten aufgelegt.

Bei der Behandlung eines Tumors im Kopf wird der Patient üblicherweise auf die Seite gelegt, da die Ausdehnung des Kopfes in dieser Richtung geringer ist als wenn der Patient auf dem Rücken oder dem Bauch liegen würde. Die Elektroden können also näher an den Tumor geführt werden. Dabei ergibt sich allerdings die Schwierigkeit, dass der Kopf durch die Schultern von der Liegefläche beabstandet ist und bei der Behandlung dadurch schief liegt. Man hilft sich dabei bislang damit, dass man zwischen Kopf und Liege ein Kissen anordnet. Dieses Kissen hat den entscheidenden Nachteil, dass es während der Behandlung verrutschen kann und der Kopf insgesamt nicht stabil liegt. Weiterhin hat das Kissen typischerweise eine unregelmäßige Form, so dass das erzeugte elektromagnetische Feld dadurch gestört werden kann oder verzerrt wird. Weiterhin wird das elektromagnetische Feld durch die Übergänge von der Elektrode zum Kissen und dem Übergang vom Kissen zum Kopf gestört.

DE2634628A1 beschreibt eine Vorrichtung zur Behandlung von Tumorgeweben im Körper.

DE2420883A1 beschreibt eine elektromedizinische Behandlungsapparatur zur Krebsheilung.

DE69407598T2 beschreibt ein Behandlungsgerät.

DE69733205T2 beschreibt eine Vorrichtung zur Behandlung von Krebsleiden.

DE2306922A1 beschreibt eine Bestrahlungsapparatur zur Behandlung von Krebs.

DE3340974A1 beschreibt ein Elektrotherapiegerät.

DE4007561A1 beschreibt eine Vorrichtung zur Erwärmung von Körpergewebe.

DE60019294T2 beschreibt eine elektrochirurgische Sonde zur Tumorbehandlung.

CN2329475Y beschreibt einen Elektrodenhalter.

US4140130A und EP0336370A2 beschreiben eine Elektrode.

Aufgabe der vorliegenden Erfindung ist es daher, die Behandlung des Kopfes mittels Elektro-Hyperthermie so zu ermöglichen, dass der Kopf stabil gelagert werden kann und das elektromagnetische Feld das Gewebe möglichst ungestört durchdringen kann.

In einer ersten Ausführungsform wird die der Erfindung zu Grunde liegende Aufgabe gelöst durch eine
Behandlungsliege geeignet für die Anwendung der Hyperthermie mit
a. einer modularen Liegefläche, bei der einzelne Module der Liegefläche ausgetauscht werden können, und
b. einer oberhalb der Liegefläche angeordneten Elektrode und
c. einem Modul der Liegefläche, in dem eine Elektrode angeordnet ist, nachfolgend als Elektrodenmodul bezeichnet, und
wobei die Liegefläche des Elektrodenmoduls wenigstens 10 cm oberhalb der übrigen Liegefläche angeordnet ist.

Diese Behandlungsliege hat den entscheidenden Vorteil, dass der Kopf des Patienten stabil auf der Liegefläche des Elektrodenmoduls gelagert werden kann und das Elektrodenmodul so ausgestaltet werden kann, dass das elektromagnetische Feld nicht gestört wird. Durch die modulare Gestaltung der Liegefläche kann im Unterschied zum Stand der Technik die individuelle Größe des Patienten und der genaue Ort des zu behandelnden Gewebes im Körper des Patienten berücksichtigt werden.

Vorzugsweise wird ein Hohlraum innerhalb des Elektrodenmoduls zumindest teilweise mit Wasser gefüllt, wobei das Wasservolumen insbesondere in einem Bereich von 3 bis 5 Liter liegt. Hierdurch kann das elektromagnetische Feld von der im Elektrodenmodul angeordneten Elektrode besonders störungs- und verlustfrei zum zu behandelnden Gewebe geführt werden.

Vorzugsweise weist der Hohlraum über wenigstens 90 % der Höhe des Hohlraums die Form eines Zylinders mit elliptischer oder kreisförmiger Grundfläche auf. Dadurch kann das elektromagnetische Feld besser geführt werden. Ecken oder Kanten im Hohlraum würden nämlich möglicherweise Spannungsspitzen erzeugen. Dabei könnte die Gefahr der Verbrennung für den Patienten bestehen.

Vorzugsweise wird der Hohlraum im Bereich der Liegefläche durch wenigstens eine Abschlussfolie abgeschlossen. Dadurch kann der Kopf besonders ruhig gelagert werden. Zudem hat die Folie den Vorteil, dass das Trägermedium Wasser für die elektromagnetische Strahlung besonders nah an das Gewebe herangeführt werden kann.

Vorzugsweise ist die Abschlussfolie mit der gegenüberliegenden Grundfläche des Hohlraums direkt oder indirekt über ein Verbindungselement verbunden. Besonders bevorzugt ist das Verbindungselement ein Stützkegel oder Stützbänder. Durch das Verbindungselement kann die Abschlussfolie noch stabiler gestaltet werden, so dass sich der Kopf des Patienten während der Behandlung noch weniger bewegt.

Vorzugsweise ist das Verbindungselement indirekt mit der Abschlussfolie verbunden, wobei an die Abschlussfolie ein Hohlzylinder aus Folie angeformt ist, an den wiederum das Verbindungselement angeformt ist. Es hat sich herausgestellt, dass das Verbindungselement durch den Hohlzylinder im Herstellungsprozess wesentlich einfacher mit der Abschlussfolie verbunden werden kann.

Vorzugsweise ist die Elektrode auf der der Liegefläche gegenüberliegenden Seite des Hohlraums angeordnet. Es hat sich herausgestellt, dass das elektromagnetische Feld so besonders gleichmäßig in den Körper eintreten kann. Besonders bevorzugt ist die Elektrode vom übrigen Hohlraum mechanisch abgeschirmt.

Vorzugsweise ist die Abschirmung mit der Elektrode verbunden, besonders bevorzugt von dieser aber elektrisch isoliert. Die Abschirmung ist vorzugsweise mit der der Liegefläche im Hohlraum gegenüberliegenden Begrenzung des Hohlraums verschraubt. Dabei kann die Begrenzung des Hohlraums die Grundplatte sein. Besonders bevorzugt ist zwischen der Abschirmung und der Grundplatte eine Metallplatte, insbesondere in Form eines Kreisringes, angeordnet. Die Abschirmung konnte so besonders einfach im Elektrodenmodul montiert werden. Vorzugsweise hat die Elektrode einen geringeren Durchmesser als die Abschirmung. Vorzugsweise befindet sich die Metallplatte in Form eines Kreisringes nur zwischen der Abschirmung und der Grundplatte.

Vorzugsweise ist die Liegefläche des Elektrodenmoduls höchstens 20 cm oberhalb der übrigen Liegefläche angeordnet. Dadurch wird sichergestellt, dass der Kopf des Patienten nicht überstreckt wird.

Die Höhe des Elektrodenmoduls liegt vorzugsweise in einem Bereich von 15 bis 30 cm.

Die Wand des Elektrodenmoduls zwischen Hohlraum und Außenwand besteht vorzugsweise zu wenigstens 90 Vol.%, insbesondere vollständig aus Schaumstoff.

Dabei handelt es sich vorzugsweise um einen Polyurethanschaumstoff.

Der Schaumstoff ist vorzugsweise viskoelastisch.

Etwaige Verstärkungselemente innerhalb des Schaumstoffes sind vorzugsweise nicht mit der Bodenplatte verbunden. Ganz besonders bevorzugt sind innerhalb des Schaumstoffes keine Verstärkungselemente vorgesehen. Dadurch kann der Kopf des Patienten besonders bequem positioniert werden.

Die Module der Behandlungsliege liegen vorzugsweise auf der übrigen Behandlungsliege auf. Die Module grenzen vorzugsweise lückenlos aneinander an. Die Breite und die Tiefe der Module können unabhängig voneinander in einem Bereich von 15 bis 40 cm, insbesondere in einem Bereich von 20 bis 30 cm liegen. Vorzugsweise ist die durch die Breite und die Tiefe der Module definierte Grundfläche der Module quadratisch.

Wenigstens unter der Hälfte der Module der Liegefläche befinden sich vorzugsweise Anschlussmöglichkeiten für das Elektrodenmodul.

Das Elektrodenmodul kann mit der erfindungsgemäßen Behandlungsliege verwendet werden und ist wenigstens 10 cm höher als die übrigen Module ausgeführt und weist insbesondere eine Höhe in einem Bereich von 15 bis 30 cm auf.

Die vorgenannten bevorzugten Ausführungsformen für das Elektrodenmodul gelten hierfür entsprechend.

Weitere praktische Ausführungsformen und Vorteile der Erfindung sind nachfolgend im Zusammenhang mit den Zeichnungen beschrieben. Es versteht sich, dass die Zeichnungen die Erfindung exemplarisch erläutern und den Schutzbereich der Erfindung nicht über die Merkmale in den Ansprüchen hinaus beschränken sollen. Es zeigen:
- Fig. 1: eine schematische Darstellung der erfindungsgemäßen Behandlungsliege
- Fig. 2: eine schematische Darstellung des Elektrodenmoduls

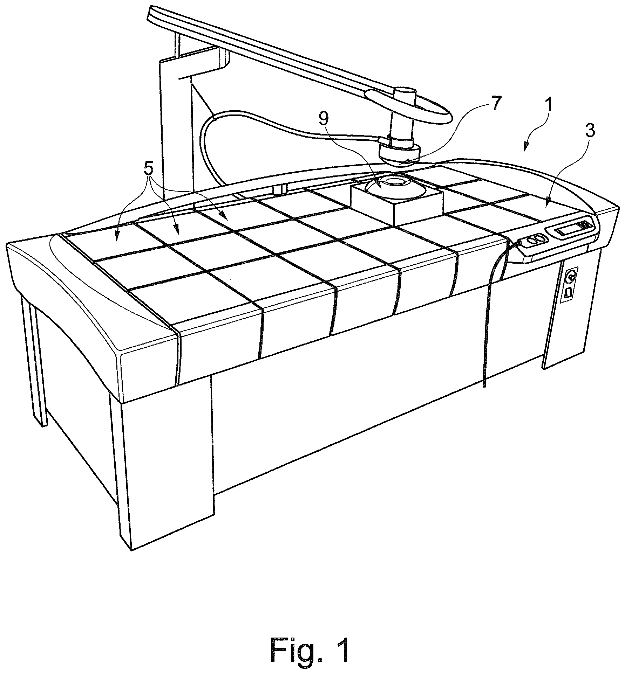

Fig. 1 zeigt die erfindungsgemäße Behandlungsliege 1 für die Anwendung der Hyperthermie mit einer modularen Liegefläche 3, bei der einzelne Module 5 der Liegefläche 1 ausgetauscht werden können, und einer oberhalb der Liegefläche 3 angeordneten Elektrode 7 und einem Elektrodenmodul 9, und wobei die Liegefläche des Elektrodenmoduls 9 wenigsten 10 cm oberhalb der übrigen Liegefläche 3 angeordnet ist.

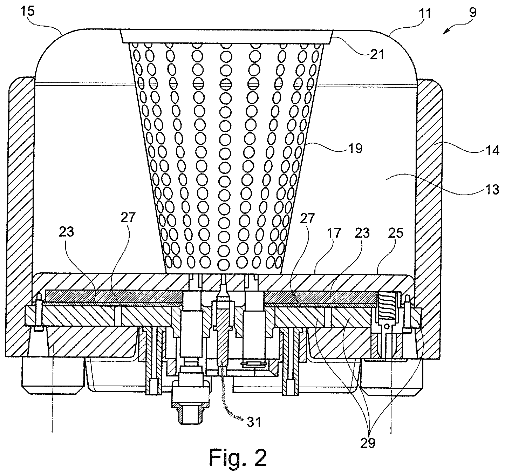

Fig. 2 zeigt das Elektrodenmodul 9 mit dem Hohlraum 13. Die Seitenwände des Hohlraums 14 bestehen in diesem Beispiel aus viskoelastischem Schaumstoff aus Polyurethan. Der Hohlraum 13 wird durch die Seitenwände des Hohlraums 14, die Grundfläche des Hohlraums 17 und die Abschlussfolie 15 bzw. die Liegefläche des Elektrodenmoduls 11 gebildet. Zwischen der Grundfläche 17 und der Abschlussfolie 15 ist ein Stützkegel als Verbindungselement 19 angeordnet, der an einem Hohlzylinder 21 befestigt ist. Der Hohlzylinder 21 ist wiederum an der Abschlussfolie 15 befestigt.

Die Elektrode 23 im Elektrodenmodul 9 ist gegenüber der Liegefläche 11 des Hohlraums 13 angeordnet. Oberhalb der Elektrode 23 ist eine Abschirmung 25 in Form eines Gitters angeordnet. Die Abschirmung 25 ist mit der Grundplatte 29 verschraubt und die Elektrode 23 darin eingefasst. Zwischen der Abschirmung 25 und der Grundplatte 29 ist eine Metallplatte 27 in Form eines Kreisringes angeordnet. Die Grundplatte 29 ist im Ausführungsbeispiel aus PVC.

Unterhalb der Grundplatte 29 sind elektrische Anschlüsse 31 vorgesehen, die die Elektrode 23 mit Spannung versorgen.

Die in der vorliegenden Beschreibung, in den Zeichnungen sowie in den Ansprüchen offenbarten Merkmale der Erfindung können sowohl einzeln als auch in beliebigen Kombinationen für die Verwirklichung der Erfindung in ihren verschiedenen Ausführungsformen wesentlich sein. Die Erfindung ist nicht auf die beschriebenen Ausführungsformen beschränkt. Sie kann im Rahmen der Ansprüche und unter Berücksichtigung der Kenntnisse des zuständigen Fachmanns variiert werden.

### Bezugszeichenliste

- 1: Behandlungsliege
- 3: Liegefläche
- 5: Module
- 7: oberhalb der Liegefläche angeordnete Elektrode
- 9: Elektrodenmodul
- 11: Liegefläche des Elektrodenmoduls
- 13: Hohlraum
- 14: Seitenwand
- 15: Abschlussfolie
- 17: Begrenzung des Hohlraums
- 19: Verbindungselement
- 21: Hohlzylinder
- 23: Elektrode im Elektrodenmodul
- 25: Abschirmung
- 27: Metallplatte
- 29: Grundplatte
- 31: Elektrische Anschlüsse

## Patentansprüche

1. Behandlungsliege (1) geeignet für die Anwendung der Hyperthermie mit
a. einer modularen Liegefläche (3), bei der einzelne Module (5) der Liegefläche (3) ausgetauscht werden können, und
b. einer oberhalb der Liegefläche (3) angeordneten Elektrode (7) und
c. einem Modul (5) der Liegefläche (3), in dem eine Elektrode angeordnet ist, nachfolgend als Elektrodenmodul (9) bezeichnet, und
wobei die Liegefläche (11) des Elektrodenmoduls (9) wenigstens 10 cm oberhalb der übrigen Liegefläche (3) angeordnet ist.

2. Behandlungsliege (1) gemäß Anspruch 1, **dadurch gekennzeichnet, dass** ein Hohlraum (13) innerhalb des Elektrodenmoduls (9) zumindest teilweise mit Wasser gefüllt ist, insbesondere das Wasservolumen in einem Bereich von 3 bis 5 Liter liegt.

3. Behandlungsliege (1) gemäß Anspruch 2, **dadurch gekennzeichnet, dass** der Hohlraum (13) über wenigstens 90 % der Höhe des Hohlraums (13) die Form eines Zylinders mit elliptischer oder kreisförmiger Grundfläche aufweist.

4. Behandlungsliege (1) gemäß einem der Ansprüche 2 bis 3, **dadurch gekennzeichnet, dass** der Hohlraum (13) im Bereich der Liegefläche (11) durch wenigstens eine Abschlussfolie (15) abgeschlossen wird.

5. Behandlungsliege (1) gemäß Anspruch 4, **dadurch gekennzeichnet, dass** die Abschlussfolie (15) mit der gegenüberliegenden Grundfläche (17) des Hohlraums (13) direkt oder indirekt über ein Verbindungselement (19) verbunden ist, insbesondere das Verbindungselement (19) Stützbänder sind.

6. Behandlungsliege (1) gemäß Anspruch 5, **dadurch gekennzeichnet, dass** das Verbindungselement (19) indirekt mit der Abschlussfolie (15) verbunden ist, wobei an die Abschlussfolie (15) ein Hohlzylinder (21) aus Folie angeformt ist, an den wiederum das Verbindungselement (19) angeformt ist.

7. Behandlungsliege gemäß einem der Ansprüche 2 bis 6, **dadurch gekennzeichnet, dass** die Elektrode (23) auf der der Liegefläche (11) gegenüberliegenden Seite des Hohlraums (13) angeordnet ist, insbesondere vom übrigen Hohlraum (13) mechanisch abgeschirmt ist.

8. Behandlungsliege (1) gemäß Anspruch 7, **dadurch gekennzeichnet, dass** die Abschirmung (25) mit der Elektrode verbunden aber insbesondere elektrisch isoliert ist, ganz besonders bevorzugt die Grundplatte 29 mit der der Liegefläche (11) im Hohlraum (13) gegenüberliegenden Begrenzung (17) des Hohlraums (13) verschraubt ist, insbesondere zwischen der Abschirmung (25) und einer Grundplatte (29) eine Metallplatte (27), insbesondere in Form eines Kreisringes, angeordnet, wobei die Abschirmung 25 die Elektrode 23 insbesondere einfasst.

9. Behandlungsliege (1) gemäß einem der Ansprüche 1 bis 8, **dadurch gekennzeichnet, dass** die Liegefläche (11) des Elektrodenmoduls (9) höchstens 20 cm oberhalb der übrigen Liegefläche (3) angeordnet ist.

## Claims

1. Treatment bed (1) suitable for the application of hyperthermia comprising
a. a modular lying surface (3) in which individual modules (5) of the lying surface (3) can be exchanged, and
b. an electrode (7) arranged above the lying surface (3), and
c. a module (5) of the lying surface (3) in which an electrode is arranged, hereinafter referred to as the electrode module (9), and
wherein the lying surface (11) of the electrode module (9) is arranged at least 10 cm above the remaining lying surface (3).

2. Treatment bed (1) according to claim 1, **characterized in that** a cavity (13) within the electrode module (9) is at least partially filled with water, in particular the water volume being in a range from 3 to 5 liters.

3. Treatment bed (1) according to claim 2, **characterized in that** the cavity (13) has the shape of a cylinder with an elliptical or circular base over at least 90% of the height of the cavity (13).

4. Treatment bed (1) according to one of claims 2 to 3, **characterized in that** the cavity (13) in the region of the lying surface (11) is closed by at least one closing film (15).

5. Treatment bed (1) according to claim 4, **characterized in that** the closing film (15) is connected to the opposite base (17) of the cavity (13) directly or indirectly via a connecting element (19), in particular the connecting element (19) being support strips.

6. Treatment bed (1) according to claim 5, **characterized in that** the connecting element (19) is indirectly connected to the closing film (15), wherein a hollow cylinder (21) made of film is integrally formed on the closing film (15), on which in turn the connecting element (19) is integrally formed.

7. Treatment bed according to one of claims 2 to 6, **characterized in that** the electrode (23) is arranged on the side of the cavity (13) opposite the lying surface (11), in particular being mechanically shielded from the remaining cavity (13).

8. Treatment bed (1) according to claim 7, **characterized in that** the shield (25) is connected to the electrode, however, is in particular electrically insulated, especially preferably the base plate (29) is screwed to the boundary (17) of the cavity (13) opposite the lying surface (11) in the cavity (13), in particular, a metal plate (27) is arranged between the shield (25) and a base plate (29), in particular in the form of a circular ring, wherein the shield (25) in particular surrounds the electrode (23).

9. Treatment bed (1) according to one of claims 1 to 8, **characterized in that** the lying surface (11) of the electrode module (9) is arranged at most 20 cm above the remaining lying surface (3).

## Revendications

1. Table de traitement (1) appropriée pour l'application de l'hyperthermie, comprenant :
a. une surface d'allongement modulaire (3), dont des modules individuels (5) peuvent être échangés, et
b. une électrode (7) disposée au-dessus de la surface d'allongement (3), et
c. un module (5) de la surface d'allongement (3), dans lequel une électrode est disposée, désigné ci-après module d'électrode (9), et
dans laquelle la surface d'allongement (11) du module d'électrode (9) est agencée au moins à 10 cm au-dessus de la surface d'allongement restante (3).

2. Table de traitement (1) selon la revendication 1, **caractérisée en ce qu'**un espace creux (13) à l'intérieur du module d'électrode (9) est rempli au moins en partie avec de l'eau, le volume d'eau étant notamment dans une plage de 3 à 5 litres.

3. Table de traitement (1) selon la revendication 2, **caractérisée en ce que** l'espace creux (13) a, au moins sur 90 % de la hauteur de l'espace creux (13), la forme d'un cylindre ayant une surface de base elliptique ou circulaire.

4. Table de traitement (1) selon l'une des revendications 2 et 3, **caractérisée en ce que** l'espace creux (13) est fermé dans la zone de la surface d'allongement (11) par au moins une feuille d'obturation (15).

5. Table de traitement (1) selon la revendication 4, **caractérisée en ce que** la feuille d'obturation (15) est reliée à la surface de base opposée (17) de l'espace creux (13) directement ou indirectement par un élément de liaison (19), l'élément de liaison (19) étant notamment des bandes de support.

6. Table de traitement (1) selon la revendication 5, **caractérisée en ce que** l'élément de liaison (19) est relié indirectement à la feuille d'obturation, et dans laquelle un cylindre creux (21) constitué d'une feuille est moulé sur la feuille d'obturation, sur lequel l'élément de liaison (19) est moulé à son tour.

7. Table de traitement selon l'une des revendications 2 à 6, **caractérisée en ce que** l'électrode (23) est disposée sur le côté de l'espace creux (23) opposé à la surface d'allongement (11), et **en ce qu'**elle est notamment blindée mécaniquement par rapport à l'espace creux (13) restant.

8. Table de traitement (1) selon la revendication 7, **caractérisée en ce que** le blindage (25) est relié à l'électrode, mais, notamment, est isolé électriquement, et de manière plus préférée, la plaque de base (29) est vissée à la limite de l'espace creux (13) opposée à la surface d'allongement (11) dans l'espace creux (13), et notamment une plaque métallique (27), particulièrement en forme d'anneau circulaire est agencée entre le blindage (25) et une plaque de base (29), et dans laquelle, en particulier, le blindage (25) borde l'électrode (23).

9. Table de traitement (1) selon l'une des revendications 1 à 8, **caractérisée en ce que** la surface d'allongement (11) du module d'électrode (9) est disposée à une hauteur maximale de 20 cm au-dessus de la surface d'allongement (3) restante.
